# EUROPÄISCHE PATENTSCHRIFT

(11) **EP 0 920 408 B1**
(45) Veröffentlichungstag und Bekanntmachung des Hinweises auf die Patenterteilung: **21.11.2001**
(21) Anmeldenummer: 97938814.7
(22) Anmeldetag: 09.07.1997
(51) Int. Cl.: C07C 57/04, C07C 51/43

(54) **Verfahren zur Herstellung von Acrylsäure oder Methacrylsäure**
Method for preparing arylic acid or methacrylic acid
Procédé pour la préparation d'acide acrylique ou d'acide methacrylique

(30) Priorität: 10.07.1996 DE 19627847
(43) Veröffentlichungstag der Anmeldung: 09.06.1999
(73) Patentinhaber: BASF AKTIENGESELLSCHAFT, 67056 Ludwigshafen (DE)
(72) Erfinder: ECK, Bernd, D-68519 Viernheim (DE); MACHHAMMER, Otto, D-68163 Mannheim (DE); PROLL, Theo, D-67098 Bad Dürkheim (DE); SCHLIEPHAKE, Volker, D-67105 Schifferstadt (DE); MÜLLER-ENGEL, Klaus, Joachim, D-76297 Stutensee (DE)
(74) Vertreter: Isenbruck, Günter, Dr.
(86) Internationale Anmeldenummer: EP9703630
(87) Internationale Veröffentlichungsnummer: WO9801415

(56) Entgegenhaltungen:
- EP-A- 0 616 998
- DE-A- 2 606 364

## Beschreibung

Die vorliegende Erfindung betrifft ein Verfahren zur Herstellung von Acrylsäure und Methacrylsäure.

Acrylsäure ist eine bedeutende Grundchemikalie. Aufgrund ihrer sehr reaktionsfähigen Doppelbindung sowie der Säurefunktion eignet sie sich insbesondere als Monomeres zur Herstellung von Polymerisaten. Von der hergestellten Menge an Acrylsäuremonomeren wird der größere Teil vor der Polymerisation - zu z.B. Klebstoffen, Dispersionen oder Lacken - verestert. Nur der kleinere Teil der hergestellten Acrylsäuremonomeren wird direkt - zu z.B. "Superabsorbern" - polymerisiert. Während im allgemeinen bei der direkten Polymerisation der Acrylsäure Monomere hoher Reinheit benötigt werden, sind die Anforderungen an die Reinheit der Acrylsäure nicht so hoch, wenn diese vor der Polymerisation verestert wird.

Es ist allgemein bekannt, daß Acrylsäure durch heterogen katalysierte Gasphasenoxidation von Propen mit molekularem Sauerstoff an im festen Aggregatzustand befindlichen Katalysatoren bei Temperaturen zwischen 200 und 400 °C zweistufig über Acrolein hergestellt werden kann (vgl. z.B. DE-A-1 962 431, DE-A-2 943 707, DE-C-1 205 502, DE-A-195 08 558, EP-A-0 257 565, EP-A-0 253 409, DE-A-2 251 364, EP-A-0 117 146, GB-B-1 450 986 und EP-A-0 293 224). Hierbei werden oxidische Mehrkomponenten-Katalysatoren z.B. auf der Basis von Oxiden der Elemente Molybdän, Bismut und Eisen (in der 1. Stufe) bzw. Molybdän und Vanadium (in der 2. Stufe) eingesetzt.

Aus DE-C-2 136 396 ist bekannt, die Acrylsäure aus den bei der katalytischen Oxidation von Propen bzw. Acrolein erhaltenen Reaktionsgasen durch Gegenstromabsorption mit einem Gemisch aus 75 Gew.-% Diphenylether und 25 Gew.-% Diphenyl abzutrennen. Weiterhin ist aus DE-A-2 449 780 das Abkühlen des heißen Reaktionsgases durch Teilverdampfen des Lösungsmittels in einem Direktkondensator (Quenchapparat) vor der Gegenstromabsorption bekannt. Problematisch ist hierbei sowie bei weiteren Verfahrensschritten der Anfall von Feststoffen in den Apparaten, der die Anlagenverfügbarkeit reduziert. Gemäß DE-A-4 308 087 kann dieser Feststoffanfall dadurch reduziert werden, indem man dem relativ unpolaren Lösungsmittelgemisch aus Diphenylether und Diphenyl ein polares Lösungsmittel wie Dimethylphthalat in einer Menge von 0,1 bis 25 Gew.-% zufügt.

Neben der oben beschriebenen Absorption des die Acrylsäure enthaltenden Reaktionsprodukts in ein hochsiedendes Lösungsmittelgemisch sehen andere bekannte Verfahren eine Totalkondensation von Acrylsäure und des weiterhin bei der katalytischen Oxidation entstehenden Reaktionswassers vor. Dabei entsteht eine wäßrige Acrylsäurelösung, die über Destillation mit einem azeotropen Mittel (vgl. DE-C-3 429 391, JP-A-1 124 766, JP-A-7 118 766, JP-A-7 118 966-R, JP-A-7 118 968-R und JP-A-7 241 885) oder über ein Extraktionsverfahren (vgl. DE-A-2 164 767, JP-A-5 81 40-039, JP-A-4 80 91 013) weiter aufgearbeitet werden kann. In EP-A-0 551 111 wird das mittels katalytischer Gasphasenoxidation hergestellte Gemisch von Acrylsäure und Nebenprodukten mit Wasser in einem Absorptionsturm in Berührung gebracht und die erhaltene wäßrige Lösung in Anwesenheit eines Lösungsmittels, das mit polaren Leichtsiedern wie Wasser oder Essigsäure ein Azeotrop bildet, destilliert. DE-C-2 323 328 beschreibt die Abtrennung von Acrylsäure aus einer angesäuerten, wäßrigen Butanol-Acrylsäure-Veresterungsablauge durch Extraktion mit einem speziellen Gemisch organischer Lösungsmittel.

Nachteilig bei den oben beschriebenen Verfahren ist, daß zur Extraktion oder Absorption ein organisches Lösungsmittel verwendet wird, das in einer weiteren Verfahrensstufe wie eine Rektifikation bei hoher thermischer Belastung wieder abgetrennt wird. Hierbei besteht die Gefahr einer Polymerisierung der Acrylsäure.

JP-A-7 082 210 beschreibt ein Verfahren zur Reinigung von Acrylsäure, die neben Acrylsäure Essigsäure, Propionsäure, Acrolein und Furfural enthält. Bei diesem Verfahren wird nach Zugabe von Wasser eine Kristallisation im Vakuum durchgeführt, wobei nach Abtrennung und Waschen der Acrylsäurekristalle eine Reinheit von 99,6% erreicht wird. Das japanische Patent 45-32417 offenbart ein Verfahren, bei dem eine wäßrige Acrylsäurelösung bzw. Methacrylsäurelösung, die zusätzlich Essigsäure und Propionsäure enthält, mit Heptan oder Toluol extrahiert wird und anschließend Wasser durch Destillation aus dem Extrakt entfernt wird. In der nächsten Stufe wird der verbleibende Extrakt auf -20 bis -80 °C abgekühlt, um eine Kristallisation von Acrylsäure bzw. Methacrylsäure herbeizuführen. Die Kristalle werden abgetrennt, und die Mutterlauge wird dem Extraktionsprozeß rückgeführt. Gemäß dieser Patentschrift ist die Verwendung eines organischen Lösungs- bzw. Extraktionsmittels notwendig, da ansonsten die Lösung, wenn sie abgekühlt wird, sich verfestigt, ohne daß Kristalle ausfallen. Nachteilig bei diesem Verfahren ist neben dem Zusatz eines organischen Lösungsmittels, daß zur Abtrennung von Wasser eine Destillation durchgeführt werden muß. Das kanadische Patent 790 625 betrifft einen weiteren Reinigungsprozeß für Rohacrylsäure durch fraktionierte Kristallisation. Dabei wird im Falle von Propionsäure als Hauptverunreinigung der Rohacrylsäure die Temperatur nicht unter die peritektische Temperatur des Systems Acrylsäure-Propionsäure abgesenkt, während im Falle von Essigsäure als Hauptverunreinigung die Temperatur nicht unter die eutektische Temperatur des Systems Acrylsäure-Essigsäure abgesenkt wird. Die zur Kristallisation eingesetzte Acrylsäure wird hierbei nach herkömmlichen Verfahren hergestellt, zum Beispiel durch Gasphasenoxidation von Propen oder Acrolein, und anschließend einer Vorreinigung durch herkömmliche bekannte Verfahren, z.B. Extraktion, unterworfen. Gemäß den Angaben der Patentschrift wird die Kristallisation der Acrylsäure vorzugsweise im wesentlichen in Abwesenheit von Wasser durchgeführt.

In EP-A-0 616 998 wird ein Verfahren zur Reinigung von Acrylsäure mittels einer Kombination von dynamischer und statischer Kristallisation beschrieben, wobei als Einsatzprodukt vorgereinigte Acrylsäure, zum Beispiel destillativ vorgereinigte Acrylsäure, verwendet wird.

Den in den obigen Dokumenten beschriebenen Verfahren ist gemeinsam, daß sie eine Vorreinigung der Acrylsäure vor der Kristallisation erfordern. Da bei der Vorreinigung in der Regel organische Lösungsmittel eingesetzt werden, die anschließend bei hoher thermischer Belastung wieder abgetrennt werden, besteht hierbei immer das Problem einer unerwünschten Polymerisation der Acrylsäure.

Aus EP-A-0 002 612, das ein Verfahren zur Reinigung von in wäßriger Lösung vorliegender Acrylsäure durch fraktionierte Kristallisation betrifft, ist der Zusatz von Salzen zur Acrylsäurelösung bekannt, um das Eutektikum Wasser-Acrylsäure aufzubrechen, das bei 63% Volumengehalt Acrylsäure liegt.

EP-A-0 675 100 beschreibt ein Verfahren zur Herstellung α, β- ungesättigter C₃-C₆-Carbonsäuren, z. B. Methacrylsäure, durch oxidative Dehydrierung der entsprechenden gesättigten C₃-C₆-Carbonsäure gefolgt von Schmelzkristallisation mit anschließend fraktionierter Destillation oder gefolgt von fraktionierter Destillation mit anschließender Schmelzkristallisation.

Die Aufgabe der vorliegenden Erfindung bestand darin, ein Verfahren zu schaffen, bei dem Acrylsäure oder Methacrylsäure ohne aufwendige Verfahrensstufen in hoher Reinheit erhalten wird.

Überraschenderweise wurde gefunden, daß Acrylsäure oder Methacrylsäure aus einem gasförmigen Produktgemisch, das einer Kondensation unterzogen wird, direkt aus der bei der Kondensation entstehenden Lösung auskristallisiert werden kann. Insbesondere wurde gefunden, daß es hierzu keiner weiteren Reinigungsstufe und keines Zusatzes von Hilfsstoffen bedarf.

Somit betrifft die Erfindung ein Verfahren zur Herstellung von Acrylsäure oder Methacrylsäure durch:
(a) Herstellung eines gasförmigen Produktgemischs, das im wesentlichen die Zusammensetzung eines Reaktionsgemischs der katalytischen Gasphasenoxidation von C₃-/C₄-Alkanen, -Alkenen, -Alkanolen und/oder -Alkanalen und/oder Vorstufen davon zu Acrylsäure oder Methacrylsäure hat, gekennzeichnet durch
(b) Kondensation des gasförmigen Produktgemischs,
(c) Kristallisation der Acrylsäure oder Methacrylsäure aus der in Stufe (b) erhaltenen Lösung und
(d) Abtrennung der erhaltenen Kristalle aus der Mutterlauge.

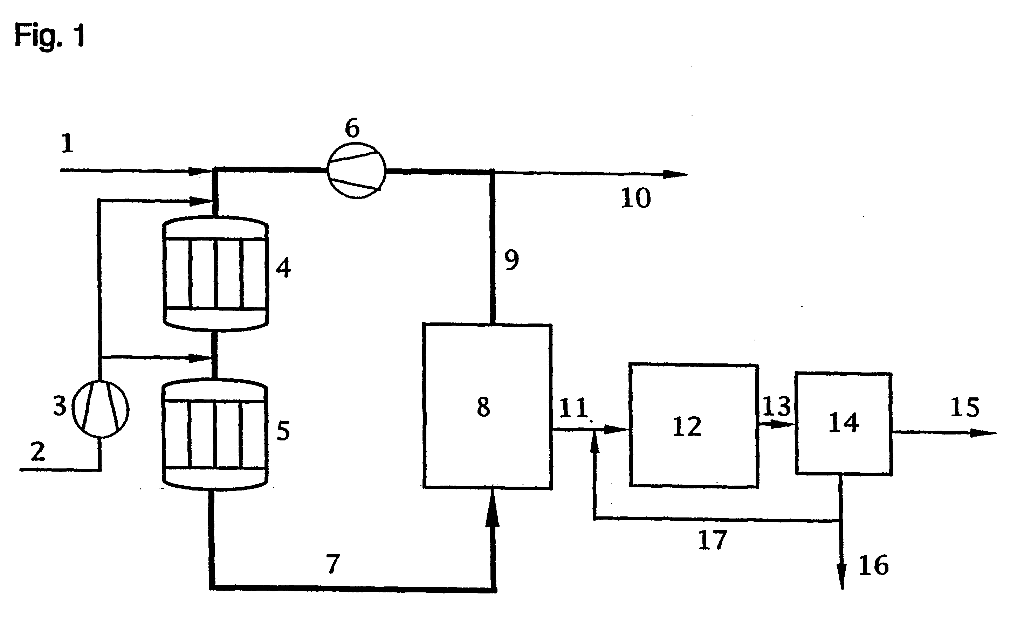

In einer bevorzugten Ausführungsform wird wenigstens ein Teil der Mutterlauge in die Stufe (c) zurückgeführt (Stufe (e)). Weitere bevorzugte Ausführungsformen der Erfindung ergeben sich aus der nachfolgenden Beschreibung, den Unteransprüchen, der Figur und dem Beispiel.

Bei dem erfindungsgemäßen Verfahren wird die Acrylsäure bzw. Methacrylsäure direkt und unmittelbar ohne weitere Zwischen- oder Reinigungsstufen und ohne Zusatz von Hilfsstoffen aus der Lösung auskristallisiert, die bei der Kondensation des Produktgemischs entsteht. Dieses Produktgemisch hat im wesentlichen die Zusammensetzung eines bei der katalytischen Gasphasenoxidation zu der Säure entstehenden Reaktionsprodukts.

Die einzige Figur zeigt ein bevorzugtes Ausführungsbeispiel zur Durchführung des erfindungsgemäßen Verfahrens.

### Stufe (a):

In Stufe (a) wird ein gasförmiges Produktgemisch hergestellt, das im wesentlichen die Zusammensetzung eines Reaktionsgemischs der katalytischen Gasphasenoxidation von C₃- bzw. C₄-Alkanen, -Alkenen, -Alkanolen und/oder -Alkanalen und/oder Vorstufen davon zu Acrylsäure oder Methacrylsäure hat. Besonders vorteilhaft wird das gasförmige Produktgemisch durch katalytische Gasphasenoxidation von Propen, Acrolein, tert.-Butanol, Isobuten, Isobutan, Isobutyraldehyd, Methacrolein, Isobuttersäure, oder Methyl-tert.-butylether hergestellt. Als Ausgangsverbindungen können alle Vorstufen der genannten Verbindungen verwendet werden, bei denen sich die eigentliche C₃-/C₄-Ausgangsverbindung erst intermediär während der Gasphasenoxidation bildet. Beispielhaft genannt für die Herstellung der Methacrylsäure sei Methyl-tert.-butylether oder Isobuttersäure.

Besonders vorteilhaft ist die katalytische Gasphasenreaktion von Propen und/oder Acrolein zu Acrylsäure mit molekularem Sauerstoff nach bekannten Verfahren, insbesondere wie sie in den oben genannten Druckschriften beschrieben sind. Vorzugsweise wird hierbei bei Temperaturen zwischen 200 und 450 °C und ggf. erhöhtem Druck gearbeitet. Vorzugsweise werden als heterogene Katalysatoren oxidische Mehrkomponenten-Katalysatoren auf der Basis der Oxide von Molybdän, Bismut und Eisen in der 1. Stufe (Oxidation von Propen zu Acrolein) und der Oxide von Molybdän und Vanadium in der 2. Stufe (Oxidation von Acrolein zu Acrylsäure) eingesetzt. Wird Propan als Ausgangsstoff verwendet, so kann dieses zu einem Propen-/Propan-Gemisch umgesetzt werden durch: katalytische Oxidehydrierung, wie z. B. in Catalysis Today 24 (1995), 307 - 313 oder US-A-5 510 558 beschrieben; durch homogene Oxidehydrierung, wie z. B. in CN-A-1 105 352 beschrieben; oder durch katalytische Dehydrierung, wie z. B. in EP-A-0 253 409, EP-A-0 293 224, DE-A-195 08 558 oder EP-A-0 117 146 beschrieben. Bei Einsatz eines Propen-/Propan-Gemischs wirkt Propan als Verdünnungsgas. Geeignete Propen-/Propan-Gemische sind auch Raffineriepropen (70 % Propen und 30 % Propan) oder Crackerpropen (95 % Propen und 5 % Propan). Grundsätzlich können Propen-/Propan-Gemische wie die o. g. mit Sauerstoff oder Luft oder einem Gemisch aus Sauerstoff und Stickstoff jeder Zusammensetzung zu Acrolein und Acrylsäure oxidiert werden.

Die Umsetzung von Propen zu Acrylsäure ist stark exotherm. Das Reaktionsgas, das neben den Edukten und Produkten vorteilhafterweise ein inertes Verdünnungsgas, z.B. Kreisgas (siehe unten), Luftstickstoff, einen oder mehrere gesättigte C₁-C₆-Kohlenwasserstoffe, insbesondere Methan und/oder Propan und/oder Wasserdampf enthält, kann daher nur einen kleinen Teil der Reaktionswärme aufnehmen. Obwohl die Art der verwendeten Reaktoren an sich keiner Beschränkung unterliegt, werden meist Rohrbündelwärmetauscher verwendet, die mit dem Oxidationskatalysator gefüllt sind, da bei diesen der überwiegende Teil der bei der Reaktion freiwerdenden Wärme durch Konvektion und Strahlung an die gekühlten Rohrwände abgeführt werden kann.

Bei der katalytischen Gasphasenoxidation wird nicht reine Acrylsäure, sondern ein gasförmiges Gemisch erhalten, das neben der Acrylsäure als Nebenkomponenten im wesentlichen nicht umgesetztes Acrolein und/oder Propen, Wasserdampf, Kohlenmonoxid, Kohlendioxid, Stickstoff, Propan, Sauerstoff, Essigsäure, Propionsäure, Formaldehyd, weitere Aldehyde und Maleinsäureanhydrid enthalten kann. Üblicherweise enthält das Reaktionsproduktgemisch, jeweils bezogen auf das gesamte Reaktionsgemisch, 1 bis 30 Gew.-% Acrylsäure, 0,05 bis 1 Gew.-% Propen und 0,05 bis 1 Gew.-% Acrolein, 0,05 bis 10 Gew.-% Sauerstoff, 0,05 bis 2 Gew.-% Essigsäure, 0,01 bis 2 Gew.-% Propionsäure, 0,05 bis 1 Gew.-% Formaldehyd, 0,05 bis 2 Gew.-% Aldehyde, 0,01 bis 0,5 Gew.-% Maleinsäureanhydrid und 20 bis 98 Gew.-%, vorzugsweise 50 bis 98 Gew.-% inerte Verdünnungsgase. Als inerte Verdünnungsgase sind insbesondere gesättigte C₁-C₆-Kohlenwasserstoffe, wie 0 bis 90 Gew.-% Methan und/oder Propan, daneben 1 bis 30 Gew.-% Wasserdampf, 0,05 bis 15 Gew.-% Kohlenoxide und 0 bis 90 Gew.-% Stickstoff, jeweils bezogen auf 100 Gew.-% Verdünnungsgas, enthalten.

Die Methacrylsäure kann analog zu Acrylsäure durch katalytische Gasphasenreaktion von C₄-Ausgangsverbindungen mit molekularem Sauerstoff hergestellt werden. Besonders vorteilhaft ist die Methacrylsäure, z. B. durch katalytische Gasphasenoxidation von Isobuten, Isobutan, tert.-Butanol, Isobutyraldehyd, Methacrolein oder Methyl-tert.-butylether erhältlich. Als Katalysatoren werden ebenfalls übergangsmetallische Mischoxidkatalysatoren (z. B. Mo, V, W und/oder Fe) verwendet. Besonders geeignete Verfahren sind solche, bei denen die Herstellung ausgehend von Methacrolein erfolgt, insbesondere dann, wenn das Methacrolein durch gasphasenkatalytische Oxidation von ten.-Butanol, Isobutan oder Isobuten oder durch Umsetzung von Formaldehyd mit Propionaldehyd gemäß EP-B-0 092 097 oder EP-B-0 058 927 erzeugt wird. Somit besteht auch die Möglichkeit, Methacrylsäure zweistufig herzustellen durch (1) Kondensation von Propionaldehyd mit Formaldehyd (in Gegenwart eines sekundären Amins als Katalysator) zu Methacrolein und (2) anschließende Oxidation des Methacroleins zu Methacrylsäure.

Ebenso wie bei der Herstellung der Acrylsäure wird nicht reine Methacrylsäure, sondern ein gasförmiges Gemisch erhalten, das neben der Methacrylsäure als Nebenkomponenten im wesentlichen nicht umgesetztes Methacrolein und/oder Wasserdampf, Kohlenmonoxid, Kohlendioxid, Stickstoff, Sauerstoff, Essigsäure, Propionsäure, weitere Aldehyde und Maleinsäureanhydrid enthalten kann. Das erfindungsgemäße Verfahren wird insbesondere dann eingesetzt, wenn das Reaktionsgemisch, 0,02 bis 2 Gew.-% Methacrolein. bezogen auf das gesamte Reaktionsgemisch und ansonsten im wesentlichen die gleichen entsprechenden Bestandteile wie bei der Herstellung der Acrylsäure enthält.

### Stufe (b):

In Stufe (b) wird das in Stufe (a) erhaltene Reaktionsprodukt einer Kondensation, insbesondere einer Partial- oder Totalkondensation, unterzogen, wobei eine Lösung erhalten wird. Die Kondensation kann nach üblichen Verfahren ein- oder mehrstufig erfolgen, und die Art der Kondensation unterliegt dabei keiner besonderen Beschränkung. Vorteilhafterweise wird die Kondensation mit einem Direktkondensator durchgeführt, wobei bereits erzeugtes Kondensat mit dem heißen gasförmigen Reaktionsprodukt in Kontakt gebracht wird. Als Apparate für die Kondensation eignen sich insbesondere Sprühwäscher, Venturiwäscher, Blasensäulen oder Apparate mit berieselten Oberflächen.

Die durch Partial- oder Totalkondensation des Reaktionsprodukts aus Stufe (a) erhaltene Mischung enthält vorzugsweise 60 bis 99,5 Gew.-% Acrylsäure bzw. Methacrylsäure, 0,1 bis 40 Gew.-% Wasser, daneben 0,1 bis 15 Gew.-% Verunreinigungen, insbesondere, jeweils bezogen auf 100 Gew.-% Kondensat, 0,01 bis 5 Gew.-% Acrolein bzw. Methacrolein, 0,05 bis 5 Gew.-% Essigsäure, 0,01 bis 5 Gew.-% Propionsäure, 0,01 bis 5 Gew.-% Formaldehyd, 0,01 bis 5 Gew.-% weitere Aldehyde und 0,01 bis 5 Gew.-% Maleinsäure. Besonders bevorzugt wird bei der Kondensation ein Gemisch erhalten, welches 93 bis 98 Gew.-% Acrylsäure bzw. Methacrylsäure, 1 bis 5 Gew.-% Wasser, daneben 0,5 bis 5 Gew.-% Verunreinigungen, insbesondere, jeweils bezogen auf 100 Gew.-% Kondensat, 0,01 bis 3 Gew.-% Acrolein bzw. Methacrolein, 0,1 bis 3 Gew.-% Essigsäure, 0,01 bis 3 Gew.-% Propionsäure, 0,01 bis 3 Gew.-% Formaldehyd, 0,01 bis 3 Gew.-% weitere Aldehyde und 0,01 bis 3 Gew.-% Maleinsäure enthält.

### Stufe (c):

In Stufe (c) wird die in Stufe (b) erhaltene Lösung, die Acrylsäure bzw. Methacrylsäure enthält, kristallisiert. Somit wird die in der Kondensationsstufe erhaltene Lösung direkt der Kristallisation zugeführt. Hierbei wird ohne Zusatz eines Lösungsmittels, insbesondere ohne Zusatz eines organischen Lösungsmittels, gearbeitet. Das verwendete Kristallisationsverfahren unterliegt keiner Beschränkung. Die Kristallisation kann kontinuierlich oder diskontinuierlich, einstufig oder mehrstufig durchgeführt werden. Vorzugsweise erfolgt die Kristallisation einstufig. In einer anderen bevorzugten Ausführungsform der Erfindung wird die Kristallisation als fraktionierte Kristallisation durchgeführt. Üblicherweise werden bei fraktionierter Kristallisation alle Stufen, die ein Kristallisat erzeugen, das reiner ist als die zugeführte Acrylsäurelösung bzw. Methacrylsäurelösung, Reinigungsstufen genannt und alle anderen Stufen Abtriebsstufen genannt. Zweckmäßigerweise werden mehrstufige Verfahren hierbei nach dem Gegenstromprinzip betrieben, bei dem nach der Kristallisation in jeder Stufe das Kristallisat von der Mutterlauge abgetrennt wird und dieses Kristallisat der jeweiligen Stufe mit dem nächsthöheren Reinheitsgrad zugeführt wird, während der Kristallisationsrückstand der jeweiligen Stufe mit dem nächstniedrigen Reinheitsgrad zugeführt wird.

Vorteilhafterweise liegt die Temperatur der Lösung während der Kristallisation zwischen -25 °C und +14 °C, insbesondere zwischen 12 °C und -5 °C. Der Feststoffgehalt im Kristallisator liegt vorteilhafterweise zwischen 0 und 80 g/100 g, bevorzugt zwischen 15 und 35 g Feststoff/100 g.

In einer vorteilhaften Ausgestaltung der Erfindung erfolgt die Kristallisation durch Kühlung von Apparatewänden oder durch Verdampfung der Lösung im Vakuum. Bei der Kristallisation durch Kühlung wird die Wärme über Kratzkühler, die mit einem Rührkessel oder einem Behälter ohne Rührwerk verbunden sind, abgeführt. Der Umlauf der Kristallsuspension wird hierbei durch eine Pumpe gewährleistet. Daneben besteht auch die Möglichkeit, die Wärme über die Wand eines Rührkessels mit wandgängigem Rührer abzuführen. Eine weitere bevorzugte Ausführungsform ;bei der Kühlungskristallisation ist die Verwendung von Kühlscheibenkristallern, wie sie z.B. von der Fa. Gouda (Holland) hergestellt werden. Bei einer weiteren geeigneten Variante zur Kristallisation durch Kühlung wird die Wärme über herkömmliche Wärmeüberträger (bevorzugt Rohrbündel- oder Plattenwärmeüberträger) abgeführt. Diese Apparate besitzen im Gegensatz zu Kratzkühlern, Rührkesseln mit wandgängigen Rührern oder Kühlkristallscheiben keine Vorrichtung zur Vermeidung von Kristallschichten auf den wärmeübertragenden Flächen. Wird im Betrieb ein Zustand erreicht, bei dem der Wärmedurchgangswiderstand durch Kristallschichtbildung einen zu hohen Wert annimmt, erfolgt die Umschaltung auf einen zweiten Apparat. Während der Betriebszeit des zweiten Apparats wird der erste Apparat regeneriert (vorzugsweise durch Abschmelzen der Kristallschicht oder Durchspülen des Apparats mit ungesättigter Lösung). Wird im zweiten Apparat ein zu hoher Wärmedurchgangswiderstand erreicht, schaltet man wieder auf den ersten Apparat um usw. Diese Variante kann auch mit mehr als zwei Apparaten im Wechsel betrieben werden. Außerdem kann die Kristallisation durch eine herkömmliche Verdampfung der Lösung im Vakuum erfolgen. In einer weiteren vorteilhaften Ausführung der Erfindung erfolgt die Kristallisation in Apparaten, in denen die Kristalle im Kristallisationsapparat an gekühlten Flächen aufwachsen, d.h. im Apparat fixiert sind (z.B. Schichtkristallisationsverfahren der Firma Sulzer Chemtech (Schweiz) oder Statisches Kristallisationsverfahren der Firma BEFS PROKEM (Frankreich)).

### Stufe (d):

In Stufe (d) werden die in Stufe (c) erhaltenen Acrylsäurekristalle bzw. Methacrylsäurekristalle von der Mutterlauge abgetrennt. Für den Fall der Schichtkristallisation oder der Statischen Kristallisation kann die Trennung der Kristalle von der Mutterlauge im Kristallisationsapparat selbst erfolgen, da die Kristalle im Apparat fixiert sind und die Mutterlauge durch Abfließenlassen aus dem Apparat entfernt werden kann. Die Entfernung der Kristalle aus dem Kristallisationsapparat erfolgt durch Aufschmelzen der Kristalle und nachfolgendes Abfließenlassen der Schmelze. Für den Fall der Suspensionskristallisation eignen sich alle bekannten Verfahren der Fest-Flüssig-Trennung. In einer bevorzugten Ausführungsform der Erfindung werden die Kristalle durch Filtrieren und/oder Zentrifugieren von der Mutteriauge abgetrennt. Vorteilhafterweise wird dem Filtrieren oder Zentrifugieren eine Voreindickung der Suspension, zum Beispiel durch Hydrozyklon(e), vorgeschaltet. Zum Zentrifugieren eignen sich alle bekannten Zentrifugen, die diskontinuierlich oder kontinuierlich arbeiten. Am vorteilhaftesten werden Schubzentrifugen verwendet, die ein- oder mehrstufig betrieben werden können. Daneben eignen sich auch Schneckensiebzentrifugen oder Schneckenaustragszentrifugen (Dekanter). Eine Filtration erfolgt vorteilhafterweise mittels Filternutschen, die diskontinuierlich oder kontinuierlich, mit oder ohne Rührwerk, oder mittels Bandfilter betrieben werden. Allgemein kann das Filtrieren unter Druck oder im Vakuum erfolgen.

Während und/oder nach der Fest-Flüssig-Trennung können weitere Verfahrensschritte zur Steigerung der Reinheit der Kristalle bzw. des Kristallkuchens vorgesehen werden. In einer besonders vorteilhaften Ausgestaltung der Erfindung schließt sich nach dem Abtrennen der Kristalle von der Mutterlauge ein ein- oder mehrstufiges Waschen und/oder Schwitzen der Kristalle oder des Kristallkuchens an. Beim Waschen liegt die Waschflüssigkeitsmenge geeigneterweise zwischen 0 und 500 g Waschflüssigkeit/100 g Kristallisat, vorzugsweise zwischen 30 und 200 g Waschflüssigkeit/100 g Kristallisat. Die verwendete Waschflüssigkeit unterliegt keiner Einschränkung. Vorteilhafterweise wird jedoch mit Reinprodukt gewaschen, d.h. mit einer Flüssigkeit, die Acrylsäure bzw. Methacrylsäure enthält, deren Reinheit höher ist als die des zu waschenden Kristallkuchens. Daneben ist auch eine Wäsche mit Wasser möglich. Das Waschen kann in hierfür üblichen Apparaten erfolgen. Vorteilhafterweise werden Waschkolonnen, in denen die Abtrennung der Mutterlauge und das Waschen in einem Apparat erfolgen, Zentrifugen, die ein- oder mehrstufig betrieben werden können, oder Filternutschen oder Bandfilter verwendet. Das Waschen kann auf Zentrifugen oder Bandfiltern ein- oder mehrstufig durchgeführt werden. Hierbei kann die Waschflüssigkeit im Gegenstrom zum Kristallkuchen geführt werden.

Beim Schwitzen handelt es sich um ein lokales Abschmelzen verunreinigter Bereiche. Vorteilhafterweise beträgt die Schwitzmenge zwischen 0 und 100 g abgeschmolzenes Kristallisat/100 g Kristallisat vor dem Schwitzen, vorzugsweise zwischen 5 und 35 g abgeschmolzenes Kristallisat/100 g Kristallisat. Besonders bevorzugt ist die Durchführung des Schwitzens auf Zentrifugen oder Bandfiltern. Auch die Durchführung einer Kombination aus Waschen und Schwitzen in einem Apparat kann geeignet sein.

Die Acrylsäurekristalle bzw. Methacrylsäurekristalle nach der Fest-Flüssig-Trennung und ggf. weiterem Waschen und/oder Schwitzen stellen die gereinigte Säure aus dem Verfahren dar. Die Reinheit der erhaltenen Kristalle beträgt in der Regel 97 bis 99,99 Gew.-% Acrylsäure bzw. Methacrylsäure, insbesondere 98,5 bis 99,9 Gew.-% Acrylsäure bzw. Methacrylsäure. Die nach dem erfindungsgemäßen Verfahren hergestellten Kristalle enthalten nurmehr ganz geringe Mengen an Verunreinigungen, wie Essigsäure, Maleinsäure oder Aldehyde.

Falls gewünscht, kann die gereinigte Säure nach bekannten Methoden ver-estert oder nach bekannten Methoden weiter gereinigt werden. In einer vorteilhaften Ausgestaltung der Erfindung schließt sich an Stufe (d) die unten erläuterte Stufe (e) an.

### Stufe (e):

In Stufe (e) wird die nach Abtrennung der Kristalle zurückbleibende Mutterlauge wenigstens teilweise direkt in die Kristallisationsstufe (c) zurückgeführt. Der Anteil der zurückgeführten Mutterlauge liegt zwischen 0 und 100 Gew.-%, vorzugsweise zwischen 20 und 80 Gew.-%.

Die Figur zeigt ein bevorzugtes Ausführungsbeispiel zur Durchführung des erfindungsgemäßen Verfahrens. Über Leitung 2 und Verdichter 3 wird den Synthesereaktoren 4 und 5 Luft zugeführt. Zusätzlich wird dem Reaktor 4 über die Leitung 9 von dem Verdichter 6 verdichtetes Kreisgas, das im wesentlichen aus Stickstoff, Kohlenoxiden und nicht umgesetzten Edukten besteht, zusammen mit aus der Leitung 1 stammendem Propen bzw. Isobuten zugeführt. Im Synthesereaktor 4 findet die erste Stufe der zweistufigen Gasphasenoxidation statt, nämlich die Oxidation von Propen bzw. Isobuten zu dem entsprechenden Acrolein. Im Synthesereaktor 5 wird dann das Acrolein zu der entsprechenden Säure oxidiert. Hierbei entsteht ein gasförmiges Produktgemisch, das neben der Säure weitere, oben genannte Verunreinigungen enthält. Dieses wird über die Leitung 7 dem Kondensator 8 zugeführt, in dem es abgekühlt und zu einer Lösung kondensiert wird. Der nichtkondensierte Teil des Produktgemischs wird über die Leitung 9 abgeführt, wobei ein Teil als Kreisgas, wie oben beschrieben, dem Reaktor 4 zurückgeführt wird und der andere Teil, vorzugsweise 50% des Gesamtstroms der Leitung 9, als Abgas aus der Anlage über die Leitung 10 abgeführt wird. Die im Kondensator 8 entstehende Lösung wird über die Leitung 11 der Kristallisationsvorrichtung 12 zugeführt, in der die Kristallisation durchgeführt wird. Die Mutterlauge aus der Kristallisation wird zusammen mit dem Kristallisat über Leitung 13 einem geeigneten Apparat 14 zur Fest-Flüssig-Trennung zugeführt, wobei über die Leitung 15 das Kristallisat und über die Leitung 16 die Mutterlauge abgeführt wird. Gemäß einer bevorzugten Ausführungsform wird wenigstens ein Teil der Mutterlauge über die Leitung 17 der Kristallisation wieder zugeführt (in Leitung 11 und Kristallisationsvorrichtung 12). Somit wird über die Leitung 15 die gereinigte Rohsäure abgeführt.

Das erfindungsgemäße Verfahren bietet gegenüber den bisher bekannten Verfahren den Vorteil, daß nach Kondensation des bei der Gasphasenoxidation entstehenden Produktgemischs direkt aus der bei der Kondensation entstehenden Lösung durch Kristallisation eine Rohsäure mit sehr guter Qualität erhalten wird. Beim Einsatz einer Kristallisation mit mehr als einer Reinigungsstufe kann direkt eine Reinsäure erzeugt werden, wobei anders als in den oben genannten Schriften, kanadisches Patent 790 625, JP-A-7 082 210 und EP-A-0 616 998 keine Vorreinigung erfolgen muß.

Ein weiterer wichtiger Vorteil des erfindungsgemäßen Verfahrens ist, daß das Verfahren relativ kalt durchgeführt wird, d.h. der Hauptstrom an Acrylsäure bzw. Methacrylsäure wird direkt über Kondensation und Kristallisation als Produkt aus dem Prozeß geführt. Da anders als im Stand der Technik kein Hilfsstoff zugesetzt wird und somit keine hohe thermische Belastung (insbesondere bei hohen Säuregehalten) zur Abtrennung dieses Hilfsstoffes erforderlich ist, werden Polymerisationsprobleme und der Einsatz von Prozeßstabilisatoren, wie sie im Stand der Technik hierbei auftreten, verringert. Außerdem wird damit auch das Fouling vermieden oder reduziert. Es ist überraschend, daß es möglich ist, durch Gasphasenoxidation und Kondensation erhaltene Acrylsäurelösungen bzw. Methacrylsäurelösungen direkt kristallisieren zu können, und daß hiermit Produkte sehr hoher Reinheit erhalten werden. Insbesondere war es überraschend, daß dies auch bei wäßrigen Kondensaten möglich ist.

Die Erfindung wird anhand des folgenden Beispiels, das eine bevorzugte Ausführungsform der Erfindung darstellt, näher erläutert.

### Beispiel

In einen 3 1 Rührbehälter aus Glas (Doppelmantel, Wendelrührer) wurden 2,3 kg des folgenden Gemisches vorgelegt: 77,8 Gew.-% Acrylsäure; 4,01 Gew.-% Essigsäure; 1,07 Gew.-% Maleinsäure; 3,66 Gew.-% Formaldehyd und 13,46 Gew.-% Wasser. Dieses Gemisch entspricht in der Darstellung der Figur dem Strom in Leitung 11, der aus dem Kondensator 8 kommt. Diese Lösung wurde durch Kühlung über den Doppelmantel des Kristallisators von Raumtemperatur auf -0,9 °C abgekühlt. Bei dieser Temperatur bildeten sich erste Acrylsäurekristalle. Die weitere Abkühlung auf -4,4 °C erfolgte in 3 h 54 min. Bei dieser Temperatur wurde ein Feststoffgehalt von 35,5 g Feststoff/100 g Suspension erhalten. 218,7 g der Suspension wurden bei -4,4 °C auf einer Zentrifuge bei 2000 U/min und einer Schleuderzeit von 1 min in 77,76 g Kristalle und 140,94 g Mutterlauge getrennt. Anschließend wurden die Kristalle mit 40 g reiner Acrylsäure mit einem Acrylsäuregehalt von mehr als 99,6% auf der Zentrifuge 1 min lang gewaschen.

Die Analyse der Kristalle ergab folgende Zusammensetzung:

| | |
|---|---|
| Acrylsäure | 98,97 Gew.-% |
| Essigsäure | 0,321 Gew.-% |
| Maleinsäure | 0,0204 Gew.-% |
| Formaldehyd | 0,119 Gew.-% |
| Wasser | 0,43 Gew.-% |

Somit führt das erfindungsgemäße Verfahren zu einer Acrylsäure, die gegenüber der eingesetzten Acrylsäure eine hohe Reinheit aufweist.

## Patentansprüche

1. Verfahren zur Herstellung von Acrylsäure oder Methacrylsäure durch:
(a) Herstellung eines gasförmigen Produktgemischs, das im wesentlichen die Zusammensetzung eines Reaktionsgemischs der katalytischen Gasphasenoxidation von C₃-/C₄-Alkanen, -Alkenen, -Alkanolen und/oder -Alkanalen und/oder Vorstufen davon zu Acrylsäure oder Methacrylsäure hat,
**gekennzeichnet durch**
(b) Kondensation des gasförmigen Produktgemischs,
(c) Kristallisation der Acrylsäure oder Methacrylsäure aus der in Stufe (b) erhaltenen Lösung und
(d) Abtrennung der erhaltenen Kristalle aus der Mutterlauge.

2. Verfahren nach Anspruch 1, **gekennzeichnet durch** die weitere Stufe (e) einer wenigstens teilweisen Rückführung der Mutterlauge aus Stufe (d) in Stufe (c).

3. Verfahren nach Anspruch 1 oder 2, **dadurch gekennzeichnet, daß** in Stufe (a) das gasförmige Produktgemisch durch katalytische Gasphasenoxidation von Propen, Acrolein, tert.-Butanol, Isobuten, Isobutan, Isobutyraldehyd, Methacrolein, Isobuttersäure oder Methyl-tert.-butylether hergestellt wird.

4. Verfahren nach einem der Ansprüche 1 bis 3, **dadurch gekennzeichnet, daß** die Kondensation als Partial- oder Totalkondensation in Stufe (b) einstufig oder mehrstufig durchgeführt wird.

5. Verfahren nach einem der vorhergehenden Ansprüche, **dadurch gekennzeichnet, daß** die Kristallisation in Stufe (c) einstufig oder mehrstufig durchgeführt wird.

6. Verfahren nach einem der vorhergehenden Ansprüche, **dadurch gekennzeichnet, daß** die Temperatur der Lösung während der Kristallisation in Stufe (c) zwischen -25 °C und +14 °C liegt.

7. Verfahren nach einem der vorhergehenden Ansprüche, **dadurch gekennzeichnet, daß** bei der Kristallisation in Stufe (c) die Wärme durch Kühlung von Apparatewänden oder durch Verdampfung der Lösung im Vakuum abgeführt wird.

8. Verfahren nach einem der vorhergehenden Ansprüche, **dadurch gekennzeichnet, daß** die Kristalle in Stufe (d) durch Filtrieren und/oder Zentrifugieren von der Mutterlauge abgetrennt werden.

9. Verfahren nach einem der vorhergehenden Ansprüche, **dadurch gekennzeichnet, daß** die in Stufe (d) abgetrennten Kristalle wenigstens einem Waschen und/oder Schwitzen unterzogen werden.

10. Verfahren nach einem der vorhergehenden Ansprüche, **dadurch gekennzeichnet, daß** in Stufe (e) zwischen 20 und 80 Gew.-% der Mutterlauge in die Stufe (c) zurückgeführt werden.

## Claims

1. A process for preparing acrylic acid or methacrylic acid, which comprises
(a)preparing a gaseous product mixture having essentially the composition of a reaction mixture of the catalytic gas phase oxidation of C₃-/C₄-alkanes, -alkenes, -alkanols and/or -alkanals and/or precursors thereof to form acrylic acid or methacrylic acid,
(b)condensing said gaseous product mixture,
(c) crystallizing acrylic acid or methacrylic acid from the solution obtained in step (b), and
(d)removing the resulting crystals from the mother liquor.

2. A process as claimed in claim 1, comprising a further step (e) of recycling at least a portion of said mother liquor from step (d) into step (c).

3. A process as claimed in claim 1 or 2, wherein said gaseous product mixture of step (a) is prepared by catalytic gas phase oxidation of propene, acrolein, tert-butanol, isobutene, isobutane, isobutyraldehyde, methacrolein, isobutyric acid or methyl tert-butyl ether.

4. A process as claimed in any of claims 1 to 3, wherein said condensing of step (b) is effected in one or more stages as a partial or total condensation.

5. A process as claimed in any of the preceding claims, wherein said crystallizing of step (c) is effected in one or more stages.

6. A process as claimed in any of the preceding claims, wherein said crystallizing of step (c) is effected at a temperature of said solution within the range from -25°C to +14°C.

7. A process as claimed in any of the preceding claims, wherein said crystallizing of step (c) is effected by removing the heat by cooling apparatus walls or by evaporating said solution under reduced pressure.

8. A process as claimed in any of the preceding claims, wherein said crystals of step (d) are removed from said mother liquor by filtration and/or centrifugation.

9. A process as claimed in any of the preceding claims, wherein said crystals removed in step (d) are subjected to at least one washing and/or sweating step.

10. A process as claimed in any of the preceding claims, wherein said recycling of step (e) is effected by recycling from 20 to 80% by weight of said mother liquor into step (c).

## Revendications

1. Procédé de fabrication de l'acide acrylique ou méthacrylique par le moyen de
a) la fabrication d'un mélange de produits gazeux étant essentiellement composé sous forme d'un mélange réactionnel de l'oxydation catalytique en phase gazeuse des alcènes, alcanols, alcanes et/ou alcanals en C₃ et C₄ et/ou leurs précurseurs pour obtenir l'acide acrylique ou méthacrylique,
**caractérisé par**
b) la condensation du mélange de produits gazeux,
c) la cristallisation de l'acide acrylique ou méthacrylique à partir de la solution obtenue à l'étape (b) et
d) la séparation des cristaux obtenus des eaux mères.

2. Procédé selon la revendication 1, **caractérisé en ce que** l'on ajoute une étape (e) dans laquelle les eaux mères de l'étape (d) sont recyclées au moins partiellement dans l'étape (c).

3. Procédé selon la revendication 1 ou 2, **caractérisé en ce que** l'on fabrique à l'étape (a) le mélange de produits gazeux au moyen de l'oxydation catalytique, en phase gazeuse, en partant de propène, d'acroléine, de t-butanol, d'isobutène, d'isobutane, d'isobutylaldéhyde, de méthacroléine, d'acide isobutyrique ou d'éther méthyl-t-butylique.

4. Procédé selon une quelconque des revendications 1 à 3, **caractérisé en ce que** l'on réalise la condensation à l'étape (b) sous forme de condensation partielle ou complète, en une étape ou en plusieurs étapes.

5. Procédé selon une quelconque des revendications précédentes, **caractérisé en ce que** l'on réalise la condensation à l'étape (c) en une étape ou en plusieurs étapes.

6. Procédé selon l'une quelconque des revendications précédentes, **caractérisé en ce que** pendant la cristallisation, la température de la solution est comprise entre -25 °C et +14 °C.

7. Procédé selon l'une quelconque des revendications précédentes, **caractérisé en ce que** lors de la cristallisation à l'étape (c), la chaleur est dissipée par refroidissement des parois des appareils ou par l'évaporation de la'solution sous vide.

8. Procédé selon une quelconque des revendications précédentes, **caractérisé en ce que** l'on sépare les cristaux à l'étape (d) des eaux mères par filtration et/ou par centrifugation.

9. Procédé selon l'une quelconque des revendications précédentes, **caractérisé en ce que** l'on soumet les cristaux séparés à l'étape (d) au moins une fois à un lavage et/ou à un ressuage.

10. Procédé selon l'une quelconque des revendications précédentes, **caractérisé en ce que** l'on recycle entre 20 % et 80 % en poids des eaux mères de l'étape (e) à l'étape (c).
